(19) Europäisches Patentamt
European Patent Office
Office européen des brevets

(11) Veröffentlichungsnummer: **0 058 928**
**B1**

(12) # EUROPÄISCHE PATENTSCHRIFT

(45) Veröffentlichungstag der Patentschrift: **30.05.84**

(51) Int. Cl.³: **C 07 C 41/54**

(21) Anmeldenummer: **82101165.7**

(22) Anmeldetag: **17.02.82**

(54) **Verfahren zur Herstellung von Acetalen des Malonaldehyds.**

(30) Priorität: **21.02.81 DE 3106576**

(43) Veröffentlichungstag der Anmeldung: **01.09.82 Patentblatt 82/35**

(45) Bekanntmachung des Hinweises auf die Patenterteilung: **30.05.84 Patentblatt 84/22**

(84) Benannte Vertragsstaaten: **BE CH DE FR GB IT LI NL**

(56) Entgegenhaltungen:
**DE - A - 821 201**
**US - A - 2 459 076**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Mangold, Dietrich, Dr., Hermann-Walker-Strasse 49, D-6903 Neckargemuend (DE)**
Erfinder: **Wahl, Josef, Bitzstrasse 25, D-6707 Schifferstadt (DE)**
Erfinder: **Aders, Wolf-Karlo, Dr., Haardtstrasse 40, D-6701 Ellerstadt (DE)**

**EP 0 058 928 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von Acetalen des Malonaldehyds durch Umsetzung von Orthoestern mit Vinylethern in Gegenwart katalytischer Mengen von $FeCl_3$.

Die Synthese der Malonaldehydacetale erfolgt vorzugsweise durch katalysierte Addition von Orthoestern an Vinylether, sie ist in der Literatur mehrfach beschrieben (Beilstein 1, IV Seite 3635).

Als Katalysatoren sind Borfluorid bzw. Borfluorid-Etherat an erster Stelle, daneben $AlCl_3$, $SnCl_2$, HF, $SO_2$ und $FeCl_3$ genannt. Präparativ verwertbare Ergebnissse sind dabei vorzugsweise bei Verwendung des stark korrosiven und abwassertoxischen $BF_3$ bzw. $BF_3$-Etherats beschrieben.

Diese Verfahren ergeben Ausbeuten an Malonaldehydacetal im besten Falle von 85% ($BF_3 \times Et_2O$ – USP 2 527 533 – Bsp. 1). Zusätzlich treten Nebenreaktionen wie die weitere Addition der gebildeten Acetale an Vinylether, beispielsweise zu Produkten vom Typ Pentaalkoxipentan, auf.

Unerwähnt bleibt bei all diesen Arbeiten eine höchst unerwünschte Nebenreaktion:

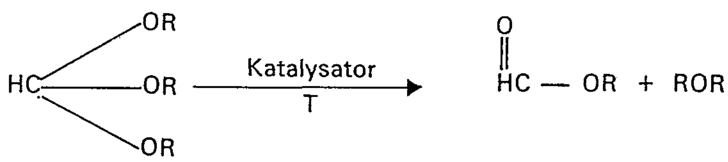


Die Fragmentierung der Orthoester zu einfachen Estern und Dialkylethern ist an sich ebenfalls bekannt (Rec. 49, 499 (1930)). Es wird gezeigt, dass diese Zersetzung und die WB/P Etherbildung insbesondere mit $FeCl_3$ als Katalysator erfolgen.

Sie führt in der Regel zu drastischen Ausbeuteverminderungen durch Verbrauch der teueren Orthoesterkomponente und vor allem zu dem unter Bedingungen der Technik sehr problematischen Anfall von leicht entflammbaren Ethern. Diese Nebenreaktion hängt in eindeutiger Weise von der Menge des vorhandenen Lewissäurekatalysators ab.

Sowohl unter Gesichtspunkten der Wirtschaftlichkeit wie auch solchen von Nebenprodukteanfall und sicherheitstechnisch einfacher Durchführung sind die beschriebenen Verfahren unbefriedigend.

Es wurde nun gefunden, dass man Acetale des Malonaldehyds der Formel

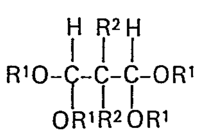

I,

worin die einzelnen Reste $R^1$ und $R^2$ gleich oder verschieden sein können und jeweils einen aliphatischen, cycloatiphatischen, araliphatischen oder aromatischen Rest bedeuten, $R^2$ auch für ein Wasserstoffatom steht, vorteilhaft erhält, wenn man Orthoester der Formel

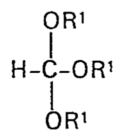

II,

worin $R^1$ die vorgenannte Bedeutung besitzt, mit Vinylethern der Formel

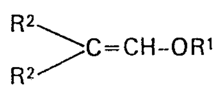

III,

worin $R^1$ und $R^2$ die vorgenannten Bedeutungen besitzen, in Gegenwart von Eisen(III)chlorid in einer Menge von höchstens 0,1 Mol je Mol Ausgangsstoff II bei Temperaturen von 0 bis 70°C umsetzt.

Die Umsetzung kann für den Fall der Verwendung von Orthoameisensäuretriethylester und Vinylmethylether durch die folgende Formelgleichung wiedergegeben werden:

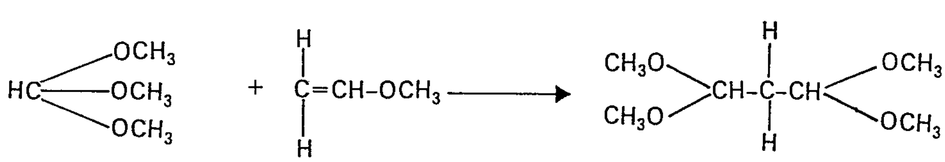

Im Vergleich zu den bekannten Verfahren liefert das Verfahren nach der Erfindung Acetale des Malonaldehyds auf einfacherem und wirtschaftlicherem Wege in besserer Ausbeute und Reinheit. Der Anfall von Nebenprodukten, z.B. von einfachen Estern und Ethern wird in weit stärkerem Masse vermieden und somit im Hinblick auf Toxizität, sicheren Betrieb und Einsparung von Kontroll- und Regelapparaturen bessere Gesamtergebnisse erzielt. Alle diese Ergebnisse sind mit Bezug auf den Stand der Technik überraschend. Ein wesentlicher Vorteil des vorliegenden Verfahrens ist auch darin zu sehen, dass die günstigen Ergebnisse bei stark reduziertem Einsatz der teuren Orthoesterkomponente erreicht werden.

Als weiterer Vorteil des vorliegenden Verfahrens zeigt sich, dass sich eine chemische Desaktivierung des Katalysators erübrigt und das Reaktionsgemisch ohne weitere Massnahme direkt nach der Umsetzung aufgearbeitet werden kann.

Die Ausgangsstoffe II und III können in stöchiometrischer Menge oder im Überschuss, vorteilhaft in einer Menge von 1 bis 3, insbesondere 1 bis 1,5 Mol Ausgangsstoff II je Mol Ausgangsstoff III umgesetzt werden. Bevorzugte Ausgangsstoffe II und III und dementsprechend bevorzugte Endstoffe I sind solche, in deren Formeln die einzelnen Reste $R^1$ und $R^2$ gleich oder verschieden sein können und jeweils einen Alkylrest mit 1 bis 12 Kohlenstoffatomen, einen Cycloalkylrest mit 5 bis 8 Kohlenstoffatomen, einen Aralkylrest oder Alkylarylrest mit 7 bis 12 Kohlenstoffatomen oder einen Phenylrest bedeuten, $R^2$ auch für ein Wasserstoffatom stehen kann. Die vorgenannten Reste können auch durch unter den Reaktionsbedingungen inerte Gruppen, z.B. Alkylgruppen oder Alkoxygruppen mit jeweils 1 bis 4 Kohlenstoffatomen, substituiert sein.

Es kommen z.B. folgende Ausgangsstoffe II in Betracht: Trimethyl, Triethyl-, Tripropyl-, Tributyl-, Triisobutyl-, Tri-sec-butyl-, Tri-tert.-butyl-, Tripentyl-, Trihexyl-, Triheptyl-, Trioctyl-, Trinonyl-, Tridecyl-, Triundecyl-, Tridodecyl-, Phenyl-, Methyldiethyl-, Methylethylpropyl-, Cyclohexyl-, Benzyl-orthoester der Ameisensäure.

Als Ausgangsstoffe III kommen in Frage: Methyl-, Ethyl-, Propyl-, Isobutyl-, Cyclohexyl-, Isoamyl-, Neopentyl-,2-Ethylhexan-1-yl-, Isopropyl-, n-Butyl-, sek.-Butyl-, tert.-Butyl-, Pentyl-, Hexyl-, Heptyl, Nonyl-, Decyl-, Undecyl-, Dodecyl-vinylether; entsprechend substituierte sowie am Vinylrest einfach oder zweifach gleich oder unterschiedlich durch die Methyl-, Ethyl-, Propyl-, Isopropyl-, Butyl-, Isobutyl-, sek.-Butyl-, tert.-Butyl, Pentyl-, Hexyl-, Ethyl-, Octyl-, Cyclohexyl-, Benzyl-, Phenyl-Gruppe substituierte Vinylether.

Die Umsetzung wird bei einer Temperatur von 0 bis 70, vorteilhaft 20 bis 50°C, drucklos oder unter Druck, kontinuierlich oder diskontinuierlich durchgeführt. Zwar kann man unter den Reaktionsbedingungen inerte organische Lösungsmittel, z.B. aromatische Kohlenwasserstoffe, verwenden, in der Regel liefern aber die Ausgangsstoffe II, III und der Endstoff I das Reaktionsmedium. Eisen-III-chlorid kann in wasserfreier oder kristallwasserhaltiger Form verwendet werden. Der Katalysator Eisen-III-chlorid wird in einer Menge von höchstens 0,1, zweckmässig von 0,0001 bis 0,1, insbesondere 0,0003 bis 0,004 Mol je Mol Ausgangstoff II verwendet.

Die Reaktion kann wie folgt durchgeführt werden: Ein Gemisch der Ausgangsstoffe II und III und Eisen-III-chlorid wird 0,25 bis 10 Stunden bei der Reaktionstemperatur gehalten. Der Endstoff wird in üblicher Weise, z.B. durch Destillation, isoliert. Bei der kontinuierlichen Herstellung von Endstoff I kann der Katalysator auf einen festen Träger adsorbiert oder aber gelöst in Orthoester oder Reaktionsgemisch zusammen mit den Reaktanten dem Reaktor zugeführt werden.

Die nach dem Verfahren der Erfindung herstellbaren Malonaldehydacetale I sind wertvolle Zwischenprodukte bei der Herstellung von Farbstoffen, Pflanzenschutzmitteln und Pharmazeutika. Bezüglich der Verwendung wird auf die vorstehend genannte Literatur verwiesen.

Die in den folgenden Beispielen angegebenen Teile bedeuten Gewichtsteile. Die Gewichtsteile verhalten sich zu den Volumenteilen wie Kilogramm zu Liter.

Beispiel 1

In einem Rührgefäss mit aufgesetzter Kolonne werden 65 000 Teile Orthoameisensäuretrimethylester vorgelegt und mit 30 Teilen $FeCl_3$ (wasserfrei) versetzt. Bei 35–40°C wird unter Rühren und Kühlen Vinylmethylether (28 000 Teile) zugefügt. Anschliessend wird mit der Abdestillation des überschüssigen Orthoesters bei Normaldruck begonnen (13 155 Teile werden zurückgewonnen).

Durch Vakuumrektifikation gewinnt man 74 345 Teile Tetramethoxipropan (Rheinheit 99%) $Kp_{20}$ 58–62°C, entsprechend einer Ausbeute von 92% (bez. auf Orthoester).

Beispiel 2

In einem Rührgefäss werden 592 Teile Orthoameisensäuretriethylester mit 2 Teilen $FeCl_3$ (wasserfrei) versetzt, anschliessend werden 216 Teile Ethylvinylether bei 38–42°C zugegeben.

Bei reduziertem Druck wird anschliessend überschüssiger Orthoester abdestilliert ($Kp_{20\,mbar}$: 45–52°C) (133 Teile). Durch Vakuumrektifikation gewinnt man 607 Teile Tetraethoxipropan (Reinheit 98,5%) entsprechend einer Ausbeute von 89% (bez. auf Orthoester). Kp. 0,2 60–62°C.

Beispiel 3

Analog Beispiel 1 versetzt man 392 Teile Orthoameisensäuretriethylester mit 0,2 Teilen $FeCl_3$ und fügt anschliessend 205 Teile Propenylethylether zu. Die Destillation über eine Vigreux-Kolonne liefert 37 Teile überschüssigen Orthoester und 502 Teile 2-Methyl-1,1,3,3-tetraethoxipropan $Kp_{20\,mbar}$: 88–92°C (Ausbeute bez. auf Ortho-Ester 90%).

Beispiel 4

In ein 100 Volumenteile fassendes, mit Glaskugeln gefülltes Reaktionsrohr wird über eine Dosierpumpe eine Lösung von 0,02 Teilen $FeCl_3$ in 100 Teilen Trimethylorthoformiat mit einer Menge von 112 Teilen/h eingeleitet und gleichzeitig über ein Regulierventil Vinylmetylether (31,5 Teile/h) am unteren Reaktorende so eingegast, dass kein Abgas anfällt und vollständiger Vinyletherumsatz gewährleistet ist. Reaktionstemperatur 40°C.

Der Reaktionsaustrag von 2 Betriebsstunden wird entsprechend Beispiel 1 aufgearbeitet. Neben 114 Teilen überschüssigem Orthoester erhält man 165 Teile Tetramethoxipropan $Kp_{20}$: 58–62°C (Reinheit 97%) (Ausbeute bez. auf umgesetzten Orthoester 92% der Therorie).

Beispiel 5

Man setzt analog Beispiel 1 636 Teile Trimethyl-

orthoformiat, enthaltend 200 ppm $FeCl_3$, bei 35–40°C mit 320 Teilen Vinylisobutylether um.

Man erhält durch Destillation 292 Teile Trimethylorthoformiat zurück und 607 Teile 1,1,3-Trimethoxi-3-isobutoxipropan ($Kp_{20}$: 77–83°C als Endstoff I).

**Patentanspruch**

Verfahren zur Herstellung von Acetalen des Malonaldehyds der Formel

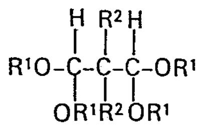

I,

worin die einzelnen Reste $R^1$ und $R^2$ gleich oder verschieden sein können und jeweils einen aliphatischen, cycloaliphatischen, araliphatischen oder aromatischen Rest bedeuten, $R^2$ auch für ein Wasserstoffatom steht, dadurch gekennzeichnet, dass man Orthoester der Formel

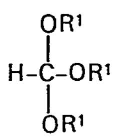

II,

worin $R^1$ die vorgenannte Bedeutung besitzt, mit Vinylethern der Formel

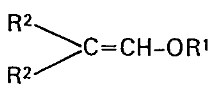

III,

worin $R^1$ und $R^2$ die vorgenannten Bedeutungen besitzen, in Gegenwart von Eisen-(III)-chlorid in einer Menge von höchstens 0,1 Mol je Mol Ausgangsstoff II bei Temperaturen von 0 bis 70°C umsetzt.

**Claim**

A process for the preparation of an acetal of malonaldehyde of the formula

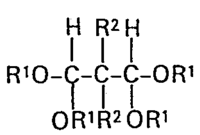

I,

where the individual radicals $R^1$ and $R^2$ can be identical or different and each is an aliphatic, cycloaliphatic, araliphatic or aromatic radical, and $R^2$ can also be hydrogen, wherein an orthoester of the formula

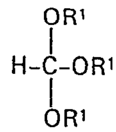

II,

where $R^1$ has the above meanings, is reacted with a vinyl ether of the formula

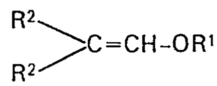

III,

where $R^1$ and $R^2$ have the above meanings, in the presence of not more than 0.1 mole of iron(III) chloride per mole of starting material II, at from 0 to 70°C.

**Revendication**

Procédé de préparation d'acétals de l'aldéhyde malonique de la formule

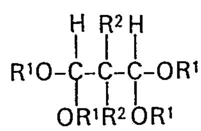

I,

dans laquelle les divers substituants $R^1$ et $R^2$ peuvent être identiques ou différents et désigner chacun un reste aliphatique, cycloaliphatique, araliphatique ou aromatique, $R^2$ puovant en outre désigner un atome d'hydrogène, caractérisé en ce que l'on fait réagir des ortho-esters de la formule

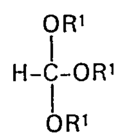

II,

dans laquelle $R^1$ possède la signification définie, et des éthers vinyliques de la formule

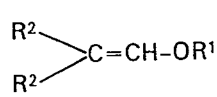

III,

dans laquelle $R^1$ et $R^2$ possèdent les significations définies plus haut, à des températures de 0 à 70°C en présence de chlorure ferrique en une proportion ne dépassant pas 0,1 mole par mole de composé de départ II.